# EUROPÄISCHE PATENTSCHRIFT

(11) **EP 0 736 286 B1**
(45) Veröffentlichungstag und Bekanntmachung des Hinweises auf die Patenterteilung: **25.06.2003**
(21) Anmeldenummer: 96109520.5
(22) Anmeldetag: 10.05.1994
(51) Int. Cl.: A61B 17/58

(54) **Osteosynthese-Hilfsmittel zur Versorgung subtrochanterer, pertrochanterer und Schenkelhalsfrakturen**
Osteosynthetic device for treating subtrochanteric and pertrochanteric fractures and fractures of the neck of the femur
Instrument auxiliaire pour ostéosynthèse permettant de traiter des fractures subtrochantériennes et pertrochantériennes, ainsi que des fractures du col du fémur

(30) Priorität: 01.06.1993 DE 4318150
(43) Veröffentlichungstag der Anmeldung: 09.10.1996
(62) Teilanmeldung aus: 94917609.3
(73) Patentinhaber: Intraplant AG, 6330 Cham (CH)
(72) Erfinder: Friedl, Wilhelm, 69214 Eppelheim (DE)
(74) Vertreter: Popp, Eugen, Dr.

(56) Entgegenhaltungen:
- EP-A- 0 321 170
- DE-U- 9 102 018
- FR-A- 2 668 360
- GB-A- 2 209 947
- US-A- 3 709 218

## Beschreibung

Die Erfindung betrifft ein Zielgerät für ein Osteosynthese-Hilfsmittel zur Versorgung subtrochanterer, pertrochanterer und Schenkelhalsfrakturen gemäß dem Oberbegriff des Patentanspruches.

Aus der EP 0 321 170 A1 ist ein Zielgerät bekannt, bei dem jeder Zielbohrung im distalen Bereich des Verriegelungsnagels eine Bohrungshülse mit kreisförmigem Querschnitt zugeordnet ist. Dies gilt auch für das Zielgerät gemäß der DE 91 02 018 Ul, obwohl eine der beiden Zielbohrungen eine Langloch-Querbohrung ist.

Aufgabe der vorliegenden Erfindung ist es, ein Zielgerät der eingangs genannten Art zur Verfügung zu stellen, welches bei einfacher Handhabung eine exakte Zuordnung von Zielbohrung und Bohrer bzw. Verriegelungsbolzen zum entweder proximalen Ende oder alternativ distalen Ende einer im distalen Bereich eines Verriegelungsnagels angeordneten Langloch-Querbohrung erlaubt.

Diese Aufgabe wird durch die Merkmale des einzigen Patentanspruches gelöst.

Kern der vorliegenden Erfindung ist es also, ein Zielgerät zur Verfügung zu stellen, mit dem die distalen Verriegelungsbolzen einer distalen Langloch-Querbohrung in einem Verriegelungsnagel exakt zugeordnet werden können, und zwar entweder am distalen oder am proximalen Ende der Langloch-Querbohrung.

Das erfindungsgemäße Zielgerät wird am proximalen Ende des Verriegelungsnagels unter Ausbildung einer starren Verbindung mit diesem angeschlossen. Der Anschluß erfolgt entweder durch eine Schraub- oder Rastverbindung. Des weiteren soll darauf geachtet werden, daß das Zielgerät ohne lange Hebelarme auskommt, um ein Verformen, durch Anfassen an einem lateralen Griff, zu vermeiden. Des weiteren soll das Zielgerät einstückig ausgebildet sein, um Relativbewegungen zwischen Teilen des Zielgerätes zu vermeiden, die zu Abweichungen von vorbestimmten Zielen führen können (Position der Schenkelhalsklinge einerseits und Position der distalen Verriegelungselemente andererseits). Es muß also die Zielgenauigkeit des Systems gesichert sein. Für die Positionierung der distalen Verriegelungselemente, insbesondere Verriegelungsbolzen, weist das erfindungsgemäße Zielgerät an einem im montierten Zustand sich etwa parallel zum Verriegelungsnagel und in Richtung zu dessen distalem Ende hin erstreckenden Arm mit mindestens einem der mindestens einen im distalen Bereich des Verriegelungsnagels ausgebildeten Langloch-Querbohrung zugeordnetes Langloch zur Aufnahme einer komplementären Zielbüchse auf, die an ihrem einen Ende eine Bohrung aufweist, deren Innendurchmesser geringfügig größer ist als der Außendurchmesser eines durch diese Bohrung hindurch zu führenden distalen Verriegelungsbolzens. Die Zielbüchse ist innerhalb des vorgenannten Langlochs um 180° wendbar, so daß die eine Bohrung in der Zielbüchse entweder dem proximalen oder dem distalen Ende der zugeordneten Langloch-Querbohrung im distalen Bereich des Verriegelungsnagels zustellbar ist. Die Zielbüchse wird nach Positionierung im vorgenannten Langloch des Zielgerätes mittels einer Feststellschraube oder dergleichen verblockt.

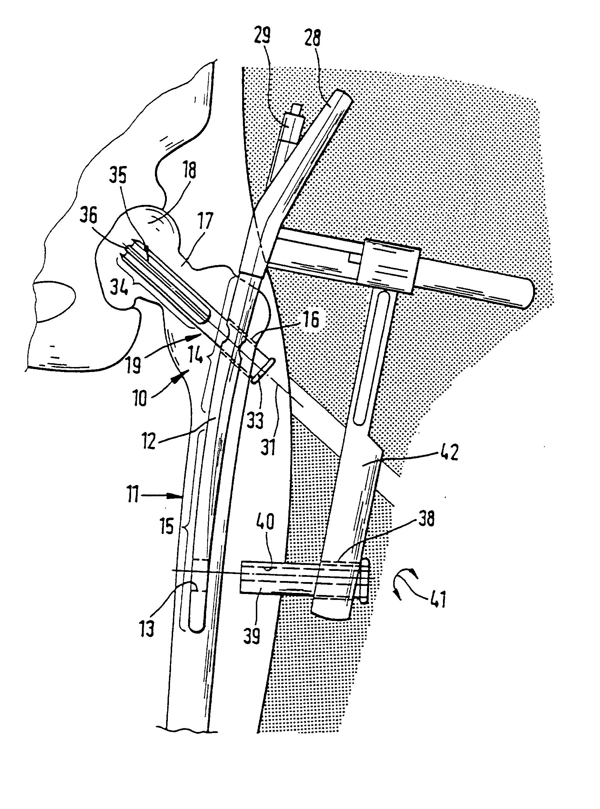

Nachstehend wird anhand der beigefügten Zeichnung eine Ausführungsform eines erfindungsgemäßen Zielgerätes näher erläutert. Die Zeichnung zeigt die Zuordnung zwischen Verriegelungsnagel, Schenkelhalsklinge und zugeordnetem Zielgerät relativ zueinander und relativ zum Femur in schematischer Vorderansicht. Das Zielgerät ist mit der Bezugsziffer 28 gekennzeichnet. Es ist am proximalen Ende eines Verriegelungsnagels 12 unter Ausbildung einer starren Verbindung mit diesem anschließbar. Es weist an einem im montierten Zustand sich etwa parallel zum Verriegelungsnagel 12 und in Richtung zu dessen distalem Ende hin mindestens ein der mindestens einen im distalen Bereich des Verriegelungsnagels 12 ausgebildeten Langloch-Querbohrung 13 zugeordnetes Langloch 38 zur Aufnahme einer komplementären Zielbüchse 39 auf, die an ihrem einen Ende eine Bohrung 40 umfaßt, deren Innendurchmesser geringfügig größer ist als der Außendurchmesser eines durch diese Bohrung hindurch zu führenden distalen Verriegelungsbolzens. Die Zielbüchse 39 ist im Langloch 38 um 180° wendbar, so daß die Bohrung 40 entweder dem proximalen oder dem distalen Ende der zugeordneten Langloch-Querbohrung 13 im distalen Bereich des Verriegelungsnagels 12 zustellbar ist. Die Möglichkeit, die Zielbüchse 39 in der beschriebenen Weise um 180° zu wenden, ist in der Zeichnung mit dem Doppelpfeil 41 angedeutet. Die Bohrung 40 dient zugleich als Bohrhülse für einen Bohrer, mit dem die Aufnahmeöffnungen im Femur für die distalen Verriegelungsbolzen vorgebohrt werden. In Verlängerung einer Schenkelhalsklinge 19 weist der vorgenannten Arm 42 einen Durchgang zur Aufnahme einer Bohr- und Zentrierhülse für die Schenkelhalsklinge auf. Diese beiden Hülsen sind in der Zeichnung nicht näher dargestellt, da es sich diesbezüglich um an sich bekannte Konstruktionselemente handelt.

Mit dem dargestellten Zielgerät wird ferner die Schenkelhalsklinge eingeführt, wobei der entsprechende Bereich im Schenkelhals und Femurkopf aufgebohrt wird, und zwar mittels eines Stufenbohrers, dessen vorderer Durchmesser etwa 3,5 mm und im übrigen etwa 10 mm beträgt, wobei der Bereich mit einem Durchmesser von 10 mm zum Aufbohren des Aufnahmeraums für den lateralen (distalen) Abschnitt der Schenkelhalsklinge dient. Für den medialen (proximalen) Profil-Abschnitt der Schenkelhalsklinge genügt eine Vorbohrung mit einem Durchmesser von etwa 3,5 mm. Die Aufbohrung erfolgt über einen Zieldraht für die Schenkelhalsklinge. Im übrigen ist das Zielgerät 28 einstückig ausgebildet, um Relativverschiebungen von einzelnen Bauteilen des Zielgerätes zu vermeiden. Dadurch wird eine hohe Zielgenauigkeit für das Einbringen der Schenkelhalsklinge sowie der distalen Verriegelungsbolzen nach Anschluß am Verriegelungsnagel 12 erreicht.

Mit der Bezugsziffer 14 ist der proximale Abschnitt des Verriegelungsnagels 12 gekennzeichnet, während der distale Abschnitt die Bezugsziffer 15 aufweist. Im proximalen Abschnitt 14 des Verriegelungsnagels ist eine schräge Durchgangsöffnung 16 vorgesehen, durch die hindurch von lateral ein Schenkelhalsteil in Form der bereits erwähnten Schenkelhalsklinge 19 in den Schenkelhals 17 und Femurkopf 18 einführbar ist. Die Schenkelhalsklinge 19 weist einen rechteckförmigen Grundquerschnitt auf. Entsprechend dem Querschnitt der Schenkelhalsklinge 19 ist der Querschnitt der schrägen Durchgangsöffnung 16 ausgebildet, wobei Schenkelhalsklinge 19 und Durchgangsöffnung 16 so relativ zueinander dimensioniert sind, daß die Schenkelhalsklinge 19 innerhalb der Durchgangsöffnung 16 spielfrei gehalten ist. In das proximale Ende des Verriegelungsnagels 12 ist ein nicht näher dargestellter Verriegelungsstift (Madenschraube oder dgl.) einsetzbar, nämlich einschraubbar. Mittels dieses Verriegelungsstiftes kann die axiale Bewegung der Schenkelhalsklinge innerhalb der schrägen Durchgangsöffnung 16 entweder begrenzt oder vollständig blockiert werden. Die rechteckförmige Durchgangsöffnung 16 ist so ausgebildet, daß die Schenkelhalsklinge 19 hochkant in der Durchgangsöffnung 16 plaziert. Die Flachseiten der Schenkelhalsklinge 19 erstrecken sich also parallel zur Längsachse des Verriegelungsnagels 12 bzw. Schenkelhalses. Diese Konstruktion führt zu einem besonders großen Biegewiderstand der Schenkelhalsklinge mit der Folge, daß keine Gefahr für ein Blockieren der Axialbewegung der Schenkelhalsklinge 19 bedingt durch eine Verbiegung derselben besteht. Bei dynamischer Plazierung der Schenkelhalsklinge 19 innerhalb der Durchgangsöffnung 16 bleibt die Dynamik sicher erhalten.

Der Verriegelungsnagel 12 weist bei der dargestellten Ausführungsform einen rotationssymmetrischen bzw. kreisrunden Querschnitt auf. Grundsätzlich ist auch ein ovaler Querschnitt denkbar, um die Rotationsstabilität innerhalb des Femur zu fördern.

Die starre Verbindung zwischen Zielgerät 28 und dem proximalen Ende des Verriegelungsnagels 12 erfolgt durch Form- und Kraftschluß, wobei der Kraftschluß durch eine Schraubverbindung (Schraubbolzen 29) hergestellt wird.

Die Schenkelhalsklinge 19 weist an ihrem äußeren, dem Femurkopf 18 abgewandten Ende einen radial vorspringenden Absatz in Form eines sich über den Umfang erstreckenden Kragens 33 auf, der die laterale Einführung der Schenkelhalsklinge 19 in die schräge Durchgangsöffnung 16 des Verriegelungsnagels 12 begrenzt. Der im Femurkopf 18 und Schenkelhals 17 verankerbare Abschnitt 34 der Schenkelhalsklinge 19 ist als Doppel-T bzw. I-Profil 35 mit einer zentralen Durchgangsbohrung 36 für einen nicht näher dargestellten, jedoch an sich bekannten Führungsdraht ausgebildet. Die vorderen im Femurkopf 18 liegenden Kanten des Profilabschnitts 34 sind vorzugsweise meißelartige Schneiden, um das Einschlagen der Schenkelhalsklinge 19 in die Spongiosa des Schenkelhalses und Femurkopfes ohne vorheriges Aufbohren der Spongiosa auf volle Breite der Schenkelhalsklinge zu erleichtern. Mit der beschriebenen Ausbildung der Schenkelhalsklinge 19 mit einem Doppel-T bzw. I-Profilabschnitt 34 läßt sich ein extrem hohes Flächenträgheitsmoment und damit eine extrem hohe Biegesteifigkeit bei minimaler Querschnittsfläche erreichen, so daß beim Einschlagen der Schenkelhalsklinge in den Schenkelhals und Femurkopf nur wenig Spongiosa verdrängt werden muß. Darüber hinaus begünstigt der Profilabschnitt 34 die Rotationsstabilität des Femurkopfes und Schenkelhalses im Verhältnis zum Femur bei Versorgung eines Schenkelhalsbruches oder einer pertrochanteren Fraktur.

Die Gesamtanordnung bestehend aus Verriegelungsnagel 12 und Schenkelhalsklinge 19 ist in der Zeichnung als Osteosynthese-Hilfsmittel 10 gekennzeichnet. Mit der Bezugsziffer 31 ist der erwähnte und im einzelnen nicht näher dargestellte Führungsdraht für die Axialbohrung 36 in der Schenkelhalsklinge 19 angedeutet.

## Patentansprüche

1. Zielgerät für ein Osteosynthese-Hilfsmittel (10) zur Versorgung subtrochanterer, pertrochanterer und Schenkelhalsfrakturen mit einem von proximal in den Markraum eines Femur (11) einführbaren Verriegelungsnagel (12), der einen distalen Bereich (15) mit mindestens zwei Querbohrungen (13) für die Aufnahme eines distalen Verriegelungselements, insbesondere Verriegelungsbolzens, sowie einen proximalen Abschnitt (14) mit einer schrägen Durchgangsöffnung (16) aufweist, durch die hindurch von lateral in den Schenkelhals (17) und Femurkopf (18) ein Schenkelhalsteil (19) einführbar ist, wobei mindestens eine Querbohrung (13) im distalen Bereich (15) des Verriegelungsnagels (12) als Langloch-Querbohrung ausgebildet ist, so daß der zugeordnete Verriegelungsbolzen zur statischen distalen Verriegelung des Verriegelungsnagels (12) am proximalen Ende und zur dynamischen distalen Verriegelung des Verriegelungsnagels (12) am distalen Ende dieser Langloch-Querbohrung (13) einführbar ist,
**dadurch gekennzeichnet, daß**
das Zielgerät am proximalen Ende (20) des Verriegelungsnagels (12) unter Ausbildung einer starren Verbindung mit diesem form- und kraftschlüssig anschließbar ist, sowie an einem im montierten Zustand sich etwa parallel zum Verriegelungsnagel (12) und in Richtung zu dessen distalem Ende hin erstreckenden Arm (42) mindestens ein der mindestens einen im distalem Bereich des Verriegelungsnagels (12) ausgebildeten Langloch-Querbohrung (13) zugeordnetes Langloch (38) zur Aufnahme einer komplementären Zielbüchse (39) aufweist, die an ihrem einen Ende eine Zielbohrung (40) aufweist, deren Innendurchmesser geringfügig größer ist als der Außendurchmesser eines durch diese Bohrung hindurch zu führenden Knochenbohrers und/oder distalen Verriegelungsbolzens, und die um 180° wendbar ist, so daß die eine Zielbohrung (40) entweder dem proximalen oder dem distalen Ende der zugeordneten Langloch-Querbohrung (13) im distalen Bereich (15) des Verriegelungsnagels (12) zustellbar ist.

## Claims

1. A directing device for an osteosynthetic aid (10) for treating subtrochanteric and pertrochanteric fractures and fractures of the femur neck, with an interlocking nail (12) which is guidable from proximally into the marrow cavity of a femur (11), the said interlocking nail having a distal region (15) with at least two transverse bore holes (13) for accommodating a distal interlocking member, in particular an interlocking bolt, and a proximal portion (14) with an oblique through-opening (16), through which a femur-retaining part (19) is introducible from laterally into the neck (17) and head (18) of the femur, at least one transverse bore (13) in the distal region (15) of the interlocking nail (12) being in the form of an elongated hole/transverse bore, with the result that the associated interlocking bolt is introducible for static distal interlocking of the interlocking nail (12) at the proximal end and for dynamic distal interlocking of the interlocking nail (12) at the distal end of this elongated hole/transverse bore (13),
**characterised in that**
the directing device is joinable with positive and frictional fit at the proximal end (20) of the interlocking nail (12), forming a rigid connection therewith, and has, on an arm (42) which, in the mounted state, extends roughly parallel to the interlocking nail (12) in the direction of its distal end, at least one elongated hole (38) associated with the at least one elongated hole/transverse bore (13) formed in the distal region of the interlocking nail (12) for the purpose of accommodating a complementary directing sleeve (39) which, at its one end, has a directing bore hole (40) the inner diameter of which is slightly larger than the outer diameter of a bone drill to be guided through this bore hole and/or of a distal interlocking bolt, and which may be turned through 180° so that the one directing bore hole (40) is advanceable to either the proximal or the distal end of the associated elongated hole/ transverse bore (13) interlocking nail (12) the distal region (15) of the interlocking nail (12).

## Revendications

1. Appareil à diriger un moyen auxiliaire d'ostéosynthèse (10) pour pourvoir des fractures du petit trochanter, du grand trochanter et du col du fémur d'un clou de verrouillage (12) à introduire, depuis le côté proximal, dans le canal médullaire d'un fémur (11), qui comporte une zone distale (15) avec au moins deux perçages transversaux (13) pour recevoir un élément de verrouillage distal, en particulier une tige de verrouillage, ainsi qu'un tronçon proximal (14) avec une ouverture de passage (16) oblique à travers laquelle on peut introduire un élément de col de fémur (19), depuis le côté, dans le col du fémur (17) et la tête du fémur (18), au moins un perçage transversal (13) étant réalisé dans la zone distale (15) du clou de verrouillage (10) sous la forme d'un perçage transversal à trou oblong, ce qui fait que la tige de verrouillage associée peut être introduite, pour le verrouillage distal statique du clou de verrouillage (10), à l'extrémité proximale et, pour le verrouillage distal dynamique du clou de verrouillage (12), à l'extrémité distale de ce perçage transversal à trou oblong (13),
**caractérisé en ce que** l'appareil à diriger peut être raccordé, par complémentarité de forme et par force, à l'extrémité proximale (20) du clou de verrouillage (12), en formant une liaison rigide avec celui-ci, et comporte, sur un bras (42) qui, à l'état monté, s'étend approximativement parallèlement au clou de verrouillage (12) et en direction de son extrémité distale, au moins un trou oblong (38) associé au perçage transversal à trou oblong, au moins unique, réalisé dans la zone distale du clou de verrouillage (12), pour recevoir une douille à diriger (39) complémentaire qui comporte, à l'une de ses extrémités, un perçage à diriger (40) dont le diamètre inférieur est légèrement supérieur au diamètre extérieur d'un foret à os et/ou tige de verrouillage distale à guider à travers ce perçage, et qui peut être tournée de 180°, ce qui fait que le perçage à diriger (40) peut être orienté soit vers l'extrémité proximale soit vers l'extrémité distale du perçage transversal à trou oblong (13) associé dans la zone distale (15) du clou de verrouillage (12).
